# EUROPEAN PATENT SPECIFICATION

(11) **EP 0 637 442 B1**
(45) Date of publication and mention of the grant of the patent: **01.12.1999**
(21) Application number: 93202328.6
(22) Date of filing: 06.08.1993
(51) Int. Cl.: A61H 15/00, A61H 23/00, A61N 5/06

(54) **Ultrasonic apparatus for health and beauty**
Ultraschallgerät für Gesundheits- und Schönheitsbehandlung
Appareil ultrasonique pour la santé et l'esthétique

(43) Date of publication of application: 08.02.1995
(73) Proprietor: ASEC CO., LTD., Nakano-ku, Tokyo (JP)
(72) Inventor: Watanabe, Tsutomu, c/o ASEC Co., Ltd., Nakano-ku, Tokyo (JP); Hamaura, Takashi, c/o ASEC Co., Ltd., Nakano-ku, Tokyo (JP); Utsumi, Masao, c/o ASEC Co., Ltd., Nakano-ku, Tokyo (JP)
(74) Representative: Jilderda, Anne Ayolt

(56) References cited:
- EP-A- 0 116 113
- DE-A- 3 241 094
- US-A- 4 823 042
- US-A- 5 176 130

## Description

### BACKGROUND OF THE INVENTION

### Field of the Invention

The present invention relates to an ultrasonic apparatus for health and beauty.

### Description of the Related Art

It is already known that ultrasonic waves are effective for health and beauty. A known apparatus for health and beauty which employs ultrasonic waves is disclosed in **US-A-4823042, upon which the preamble of claim 1 is based, and which discloses the same subject matter as JP-A-3123559.** In this known apparatus, a disk-like vibration-transmitting plate for being brought into contact with the skin. **This ultrasonic apparatus for the health and beauty comprises a main body having a grip portion; and an ultrasonic generating unit.**

However, with the apparatus of the above-descriped **US patent 4823042**, it is possible to obtain merely the effect based on ultrasonic waves, and a synergistic effect incorporating the effect of another method cannot be expected.

**The publications DE-A-3241094 and JP-U-215131 both disclose that a massage roller arranged at the end of a main body can be combined with a vibration means, however they are both provided with a rotating electric motor. Further in both the devices is the motor arranged in the handle and the vibrating energy is transmitted to the roller through a roller bearing portion.**

### SUMMARY OF THE INVENTION

The present invention has been devised in view of the above-described circumstances, and it is an object of the present invention to provide an unltasonic apparatus for health and beauty **having improved efficiency.**

To this end, in accordance with the present invention, there is provided an ultrasonic apparatus for health and beauty, **in accordance with claim 1. By arranging the ultrasonic generating unit inside a roller supported at the end of the main body a synergistic effect of ultrasonic "micro" massaging treatment is provided together with a traditional massaging effect provided by the roller.**

In addition to the above-described arrangement, the hollow cylindrical roller may be formed of a material which radiates far-infrared rays. In this case, the thermal effect of far-infrared rays can be exhibited in a synergistic manner. As such a far-infrared radiant material, one having a high emissivity is preferable, and it is possible to cite a resin such as a cullulose acetate resin.

The apparatus of the present invention having the above-described construction is used by bringing the hollow cylindrical roller into contact with the skin and rolling the same.

First, if a supply switch (not shown) is turned on and high-frequency power is supplied, the hollow cylindrical roller itself acts as an ultrasonic vibrator and imparts ultrasonic vibrations to the skin. In addition, in a state in which a mechanical massage is being imparted to the skin by causing the hollow cylindrical roller to roll on the skin, a micro-massaging effect produced by the ultrasonic vibrations is added, thereby making it possible to obtain a synergistic effect. The ultrasonic energy penetrates not only the tissues of the skin but also the deep interior of the body, and acts on and further activates various cells of the skin, nerves, lymphatic vessels, blood vessels, and muscle layers, thereby promoting metabolism and improving the massaging effect.

Furthermore, in a case where the hollow-cylindrical roller is formed of a material which radiates far-infrared rays, a thermal effect is added to the above-described effects, thereby further promoting the activation of the cells.

The above and other objects, features and advantages of the present invention will become more apparent from the following detailed description of the invention when read in conjunction with the accompanying drawings.

### BRIEF DESCRIPTION OF THE DRAWINGS

Fig. 1 is a front elevational view of an apparatus in accordance with an embodiment of the present invention;
Fig. 2 is a side elevational view of the apparatus shown in Fig. 1;
Fig. 3 is an enlarged cross-sectional view, taken along a plane including an axis, of a roller and its interior shown in Fig. 1;
Fig. 4 is a partially cutaway perspective view of the roller of the apparatus shown in Fig. 1;
Fig. 5 is a front elevational view of an apparatus illustrating features useful with the present invention;
Fig. 6 is a side elevational view of the apparatus shown in Fig. 5;
Fig. 7 is an enlarged cross-sectional view, taken along the plane including the axis, of the roller of the apparatus shown in Fig. 5;
Fig. 8 is an enlarged cross-sectional view, taken along a plane perpendicular to the axis, of the roller of the apparatus shown in Fig 5;
Fig. 9 is a front elevational view of an apparatus illustrating features useful with the present invention; and
Fig. 10 is a block diagram of the apparatus shown in Fig. 9.

### DESCRIPTION OF THE PREFERRED EMBODIMENTS

Referring now to the accompanying drawings, a description will be given of the embodiments of the present invention.

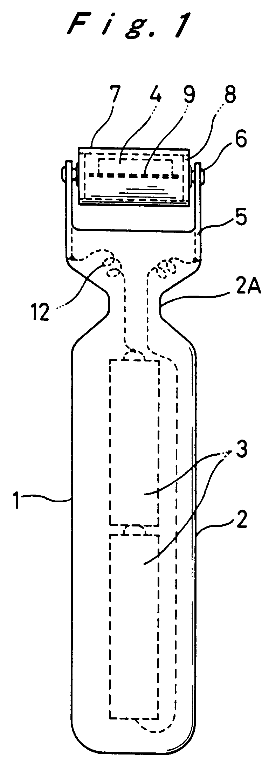

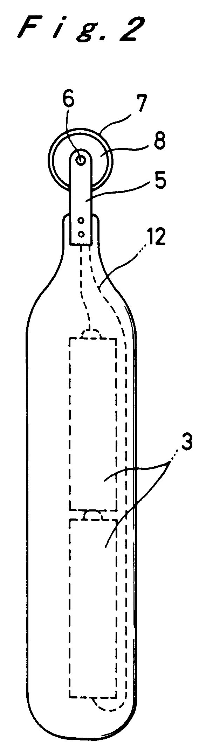

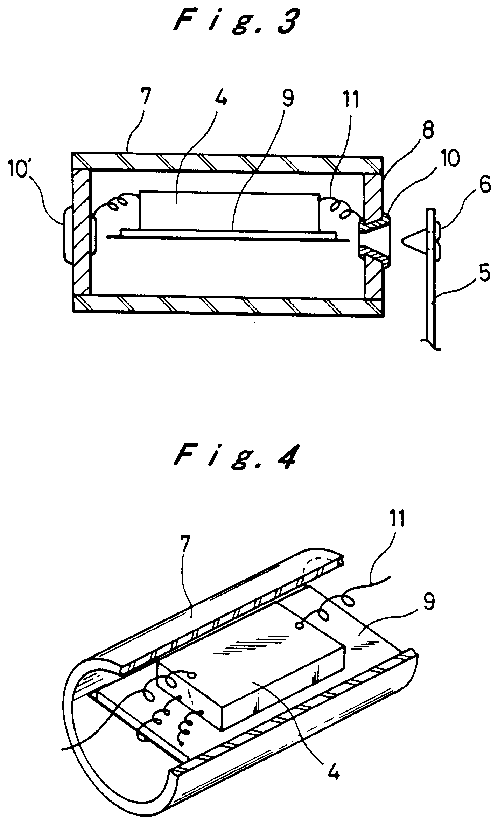

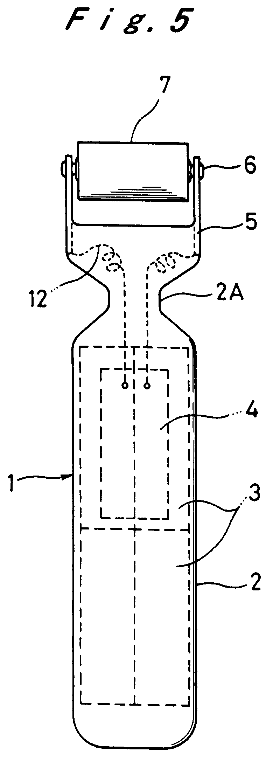

Fig. 1 is a front elevational view of an apparatus in accordance with the present invention, and Fig. 2 is a side elevational view thereof. In addition, Fig. 3 is an enlarged cross-sectional view of a roller 7, and Fig. 4 is a cutaway perspective view of the roller 7. In the drawings, reference numeral 1 denotes a main body 1 having a grip portion 2 which can be gripped by one hand. The main body 1 has an internal space, in which a power source 3 such as batteries are incorporated.

A neck portion 2A having a constricted shape is formed in an upper end portion of the main body 1, and a pair of support arms 5 formed of a metal conductor are attached to an upper end of the main body 1 in such a manner as to extend therefrom. Conical projections 6 formed of a metal conductor and constituting shafts of pivot bearings are respectively provided on distal ends of the pair of support arms 5 on opposing surfaces thereof.

The roller 7 of a hollow cylindrical shape has a pair of covers 8 respectively fitted at both ends thereof. Each conical projection 6 of the support arm 5 is fitted in a conical recessed portion 10 formed of a metal conductor and formed as a seat of the pivot bearing on an outer surface of the cover 8, so as to constitute the pivot bearing. Hence, the roller 7 is rotatable about the pivot bearings.

As shown in Fig. 3, a high-frequency generating unit 4 and an ultrasonic generating unit 9 are accommodated in the above-described roller 7. The high-frequency generating unit 4 has a circuit for generating a high frequency as electric power from the power source 3 is supplied thereto. The ultrasonic generating unit 9 is formed of a plate-like vibrator such as a piezoelectric ceramic, and the vibrator 9, which is the ultrasonic generating unit, is secured at its sides to an inner surface of the above-described roller 7. The high-frequency generating unit 4 is connected to the power source 3 via leads 11, the pivot bearings 10, 6, the support arms 5, and leads 12.

Such an apparatus in accordance with the present embodiment is used as the user, while holding the main body 1 in his or her hand, rolls the roller 7 in contact with the skin.

The vibrator 9 is driven by receiving high-frequency electric power from the high-frequency generating unit 4. The ultrasonic vibrations of the vibrator 9 are transmitted to the roller 7, and ultrasonic vibrations are produced from the roller 7.

If the ultrasonic vibrations are imparted to the skin while the roller 7 is thus being rolled, the mechanical massaging effect based on rolling and the micro-massaging effect based on the ultrasonic vibrations coact in a synergistic manner, and the effect is improved by a remarkable degree.

In the apparatus of the present embodiment, the vibrator 9 does not need to have the shape of a rectangular plate as illustrated. For instance, the vibrator 9 may be formed in the shape of a disk, and its outer peripheral surface may be bonded to the inner surface of the roller 7, or a sheet material known as a piezoelectric plastic may be attached to the inner surface of the roller 7.

Next, a description will be given of an apparatus comprising features useful with the present invention.

The present embodiment is characterized in that the roller is so arranged as to radiate far-infrared rays, and the thermal effect of far-infrared rays can be exhibited in a synergistic manner in addition to the effects based on the rolling of the roller and the ultrasonic vibrations in the foregoing embodiment.

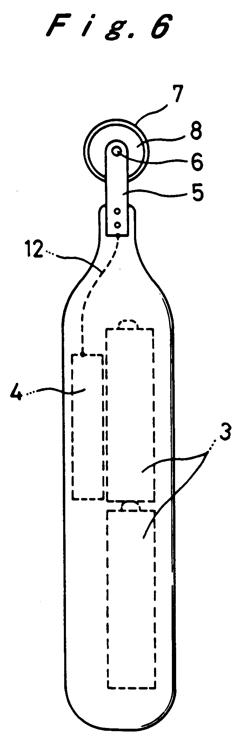

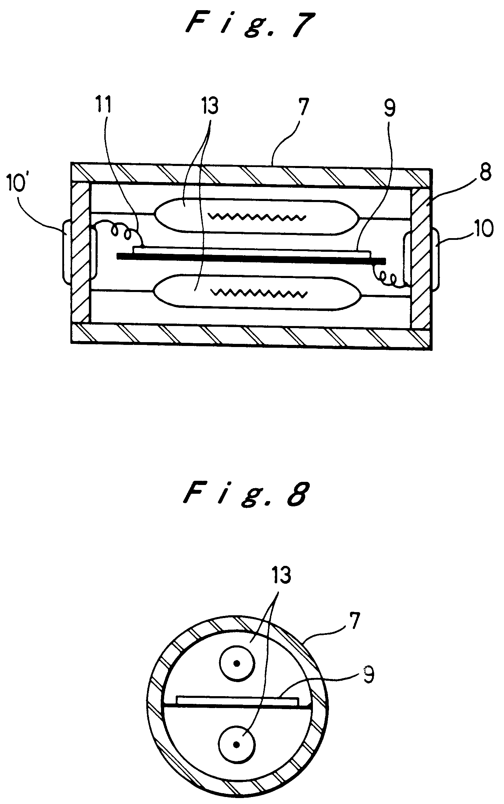

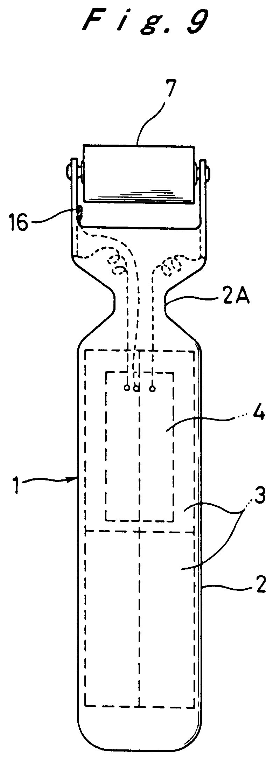

In Figs. 5, 6, 7 and 8, the roller 7 of a hollow cylindrical shape is formed of a high-polymer resin having a high emissivity of far-infrared radiation, such as a cellulose acetate resin. In addition to the vibrator 9 similar to that of the foregoing embodiment, an incandescent lamp 13 (two incandescent lamps are used in the illustrated example) serving as a heat source for heating is provided in the above-described roller 7. It should be noted that, in **an** embodiment, **in which** high-frequency generating unit 4 is accommodated in the main body **1 does not fall within the scope of the present invention.** The incandescent lamp 13 is used to heat the aforementioned roller 7 and increase the far-infrared radiant energy of the roller 7. As for the power source of the incandescent lamp 13, the high-frequency generating unit 4 in the body 1, which is used for the ultrasonic generating unit 9, is connected to the incandescent lamp 13 in parallel with the ultrasonic generating unit 9, and the high-frequency generating unit 4 is thus used to serve as a power source for the incandescent lamp.

In addition, the roller 7 may be provided with hazing and/or coloring treatment to bring about a tastewise or psychological effect at the time of use.

Next, a description will be given of an apparatus comprising further features useful with the present invention.

The present embodiment is characterized in that the temperature of the roller discussed above is maintained to a desired level to make it possible to meet the user's various demands.

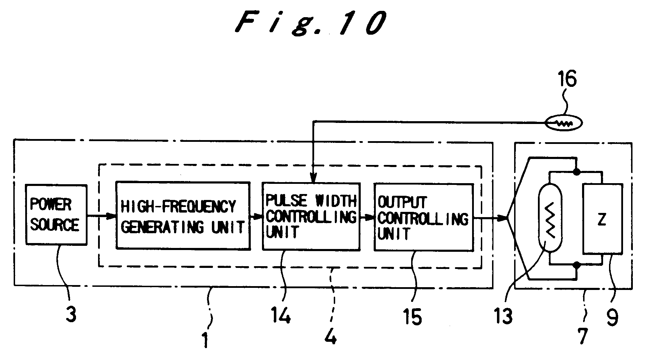

In the apparatus of the present embodiment, as shown in Figs. 9 and 10, a pulse width controlling unit 14 and an output controlling unit 15 are disposed between the high-frequency generating unit 4 on the one hand, and the ultrasonic generating unit 9 and the incandescent lamp 13 on the other. The pulse width of the generated high frequency is controlled on the basis of a signal from a temperature sensor 16 disposed on the roller 7 or in the vicinity thereof, so as to control the effective electric power supplied to the heat source, thereby allowing an optimum roller temperature to be obtained for the user and a body part to which the apparatus is applied.

As described above, in accordance with the ultrasonic apparatus for health and beauty of the present invention, a massaging effect is obtained by imparting stimuli to skin cells by applying the roller to the skin and rolling it on the skin. At the same time, since the ultrasonic generating unit is incorporated in the roller, and the roller itself functions as an ultrasonic vibrator by supplying high-frequency electric power to the ultrasonic generating unit, the micro-massaging effect can also be exhibited in a synergistic manner. Furthermore, in the case where the roller itself is formed of a material which radiates far-infrared rays, the effect of far-infrared radiation can also be obtained. Moreover, all the various functions are condensed in the hollow cylindrical roller, and the circuit system is very simple, so that the structure can be made very compact as a hand-held type or a portable type. The apparatus can be manufactured by using only those materials which are inexpensive and readily available, and the apparatus is lightweight and simple to use.

## Claims

1. An ultrasonic apparatus for health and beauty comprising:
a main body **(1)** having a grip portion **(2); and**
**an ultrasonic generating unit (9),**
**characterized in that****,**
**it also comprises** a hollow cylindrical roller **(7)** supported rotatably at one end of said main body **(1);**
**said** ultrasonic generating unit **(9) is** disposed in said hollow cylindrical roller **(7), and**
said hollow cylindrical roller **(7)** itself constitutes an ultrasonic vibrator which is exited by said ultrasonic vibrating unit **(9).**

2. An ultrasonic apparatus for health and beauty according to claim 1. wherein said hollow cylindrical roller **(7)** is formed of a material **having a high emissivity for** far-infrared rays.

3. An ultrasonic apparatus for health and beauty according to claim 2, wherein said hollow cylindrical roller **(7)** is formed of a high-polymer resin material which **has a high emissivity for** far-infrared rays at ordinary temperature.

4. An ultrasonic apparatus for health and beauty according to claim 2, further comprising: a heat source **(13)** accommodated in said hollow cylindrical roller **(7)** for heating said hollow cylindrical roller **(7)**.

5. An ultrasonic apparatus for health and beauty according to claim 4, wherein a high-frequency generating unit **(4)** for said ultrasonic generating unit **(9)** is connected to said heat source **(13)** in parallel with its connection to said ultrasonic generating unit **(9)**.

6. An ultrasonic apparatus for health and beauty according to claim 4 or 5, further comprising:
a temperature sensor disposed on said hollow cylindrical **roller (7)** or in a vicinity thereof; and
a control unit for maintaining said hollow cyclindrical roller **(7)** at a predetermined temperature on the basis of an output signal from said temperature sensor.

7. An ultrasonic apparatus for health and beauty according to claim 6, wherein said control unit varies a generated-pulse width of said high-frequency generating unit **(4)** on the basis of the output signal from said temperature sensor so as to control effective electric power supplied to said heat source **(13)**, thereby maintaining said hollow cylindrical roller **(7)** at the predetermined temperature.

8. An ultrasonic apparatus for health and beauty according to claim 4, wherein said heat source **(13)** is constituded by an incandescent lamp **(13)**, and at least one of hazing means and coloring means is provided in a vicinity of said incandescent lamp **(13)** inside said hollow cylindrical roller **(7)**.

## Patentansprüche

1. Ein Ultraschallgerät für Gesundheits- und Schönheitsbehandlung, bestehend aus:
einem Hauptkörper (1) mit einem Griffteil (2) und einer Ultraschall erzeugenden Einheit (9),
dadurch gekennzeichnet, daß es auch eine hohle zylinderische Rolle (7) umfaßt, die an dem einen Ende des besagten Körpers (1) drehbar gelagert ist;
daß besagte Ultraschall erzeugende Einheit (9) in der besagten hohlen zylindrischen Rolle (7) untergebracht ist und
daß besagte hohle zylindrische Rolle (7) selbst einen Ultraschallvibrator darstellt, der von der besagten Ultraschall erzeugenden Einheit (9) erregt wird.

2. Ein Ultraschallgerät für Gesundheits- und Schönheitsbehandlung nach Anspruch 1, wobei die besagte hohle zylindrische Rolle (7) von einem Material mit einem hohen Emissionsvermögen im fernen Infrarotstrahlungsbereich gebildet wird.

3. Ein Ultraschallgerät für Gesundheits- und Schönheitsbehandlung nach Anspruch 2, wobei die besagte hohle zylindrische Rolle (7) von einem hochpolymeren Kunstharz gebildet wird, das bei normaler Temperatur ein hohes Emissionsvermögen im fernen Infrarotstrahlungsbereich besitzt.

4. Ein Ultraschallgerät für Gesundheits- und Schönheitsbehandlung nach Anspruch 2, das ferner eine Wärmequelle (13) umfaßt, die in der besagten hohlen zylindrischen Rolle (7) zur Erwärmung der besagten hohlen zylindrischen Rolle (7) untergebracht ist.

5. Ein Ultraschallgerät für Gesundheits- und Schönheitsbehandlung nach Anspruch 4, wobei eine Einheit (4) zur Erzeugung hochfrequenter Ströme für die besagte Ultraschall erzeugende Einheit (9) mit der besagten Wärmequelle (13) parallel mit ihrer Verbindung mit der besagten Ultraschall erzeugenden Einheit (9) verbunden ist.

6. Ein Ultraschallgerät für Gesundheits- und Schönheitsbehandlung nach Anspruch 4 oder 5, das ferner einschließt:
einen Temperaturfühler, der an oder in der Nähe der hohlen zylindrischen Rolle (7) angeordnet ist, und
eine Regeleinrichtung, um besagte hohle zylindrische Rolle (7) aufgrund eines Ausgangssignals des besagten Temperaturfühlers auf einer vorbestimmten Temperatur zu halten.

7. Ein Ultraschallgerät für Gesundheits- und Schönheitsbehandlung nach Anspruch 6, wobei die besagte Regeleinrichtung eine erzeugte Impulsbreite der besagten Einheit (4) zur Erzeugung hochfrequenter Ströme aufgrund des Ausgangssignals des besagten Temperaturfühlers verändert, um so die der besagten Wärmequelle (13) zugeführte effektive elektrische Energie zu regeln, und dabei die besagte hohle zylindrische Rolle (7) auf der vorbestimmten Temperatur zu halten.

8. Ein Ultraschallgerät für Gesundheits- und Schönheitsbehandlung nach Anspruch 4, wobei besagte Wärmequelle (13) von einer Glühlampe (13) gebildet wird und mindestens eines der Trübungs- und Farbmittel in der Nähe der besagten Glühlampe (13) im Inneren der besagten hohlen zylindrischen Rolle (7) bereitgestellt wird.

## Revendications

1. Appareil à ultrasons pour la santé et l'esthétique comprenant :
un corps principal (1) comportant une partie de prise (2), et
une unité de production d'ultrasons(9),
caractérisé en ce
qu'il comprend également un rouleau cylindrique creux (7) supporté de manière à pouvoir tourner à une extrémité dudit corps principal (1);
ladite unité de production d'ultrasons(9) étant disposée dans ledit rouleau cylindrique creux (7), et
ledit rouleau cylindrique creux (7) constituant lui-même un vibrateur à ultrasons qui est excité par ladite unité à vibrations par ultrasons (9).

2. Appareil à ultrasons pour la santé et l'esthétique suivant la revendication 1, dans lequel ledit rouleau cylindrique creux (7) est formé d'un matériau présentant un pouvoir émissif élevé pour les rayons infrarouges lointains.

3. Appareil à ultrasons pour la santé et l'esthétique suivant la revendication 2, dans lequel ledit rouleau cylindrique creux (7) est formé d'un matériau à base de résine de haut polymère qui présente un pouvoir émissif élevé pour les rayons infrarouges lointains à température ordinaire.

4. Appareil à ultrasons pour la santé et l'esthétique suivant la revendication 2, comprenant en outre : une source de chaleur (13) logée dans ledit rouleau cylindrique creux (7) pour chauffer ledit rouleau cylindrique creux (7).

5. Appareil à ultrasons pour la santé et l'esthétique suivant la revendication 4, dans lequel une unité de production haute fréquence (4) pour ladite unité de production d'ultrasons (9) est reliée à ladite source de chaleur (13) parallèlement à sa connexion à ladite unité de production d'ultrasons (9).

6. Appareil à ultrasons pour la santé et l'esthétique suivant la revendication 4 ou 5, comprenant en outre :
un capteur de température disposé sur ledit rouleau cylindrique creux (7) ou à proximité de celui-ci, et
une unité de commande pour maintenir ledit rouleau cylindrique creux (7) à une température prédéterminée sur la base d'un signal de sortie provenant dudit capteur de température.

7. Appareil à ultrasons pour la santé et l'esthétique suivant la revendication 6, dans lequel ladite unité de commande fait varier une largeur d'impulsion produite de ladite unité de production haute fréquence (4) sur la base du signal de sortie provenant dudit capteur de température de manière à commander la puissance électrique effective fournie à ladite source de chaleur (13), maintenant de ce fait ledit rouleau cylindrique creux (7) à la température prédéterminée.

8. Appareil à ultrasons pour la santé et l'esthétique suivant la revendication 4, dans lequel ladite source de chaleur (13) est constituée d'une lampe à incandescence (13), et au moins un parmi des moyens de voile ou de coloration est fourni à proximité de ladite lampe à incandescence(13) à l'intérieur dudit rouleau cylindrique creux (7).
